# EUROPEAN PATENT APPLICATION

(11) **EP 4 541 903 A2**
(43) Date of publication of application: **23.04.2025**
(21) Application number: 24220945.0
(22) Date of filing: 25.03.2016
(51) Int. Cl.: C12P 19/24

(54) **FRUCTOSE TO ALLULOSE CONVERSION**

(30) Priority: 27.03.2015 US 201562139072 P
(62) Divisional of application: 16773840.0
(71) Applicant: Archer Daniels Midland Company, Decatur, IL 62526 (US)
(72) Inventor: VENKITASUBRAMANIAN, Padmesh, Forsyth, 62535 (US)
(74) Representative: dompatent von Kreisler Selting Werner - Partnerschaft von Patent- und Rechtsanwälten mbB

(57) **Abstract**

The present disclosure describes a method of producing allulose from fructose with a novel psicose-3-epimerase enzyme from a *Burkholderia* species. Once identified and isolated, the psicose-3-epimerase gene was cloned into a novel production strain and evaluated in both benchtop and pilot scale production environments. Evaluation of the in vivo enzyme activity and downstream processing involves immobilization of the enzyme on solid matrix resins, which is disclosed herein.

## Description

### CROSS REFERENCE TO RELATED APPLICATION[S]

This Application claims priority to US provisional application No. 62/139,072 entitled "FRUCTOSE TO ALLULOSE CONVERSION" filed March 27, 2015, which is incorporated herein by reference in its entirety.

### BACKGROUND OF THE INVENTION

D-Allulose is the C-3 epimer of D-fructose and is a low-caloric sweetener. Allulose, also widely known as D-psicose, is very similar to glucose in regards to intensity and sweetness. However, because the body metabolizes allulose differently than most sugars, such as glucose and fructose, its caloric value is significantly lower. In fact, its caloric value is nearly zero. Like glucose, D-allulose has about 70 % of the relative sweetness of sucrose but only provides 0.2 kcal/mol energy.

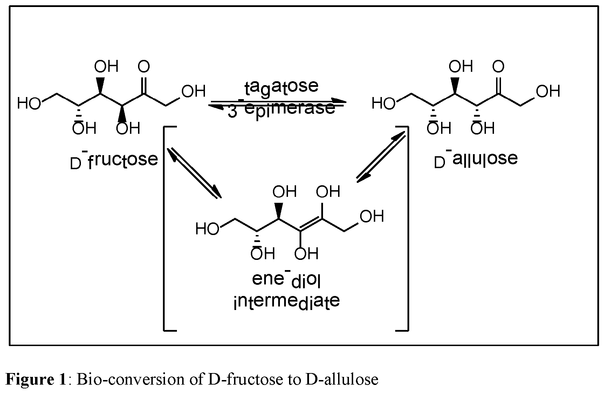

The bio-conversion of D-fructose to D-allulose by D-tagatose-3-epimerase (DT3E) or by D-psicose-3-epimerase (Figure 1) has long been recognized, however, the methods of production have typically come with high production cost. The conversion of D-fructose to D-allulose will diversify the traditional sweetener product portfolio associated with corn processing by adding a natural low caloric sweetener and bulking agent to the traditional portfolio of sweeteners derived from corn starch, *i.e.* com syrup, high fructose com syrup (HFCS), glucose and fructose.

US Patent Application No: 20150210996 (Woodyer et al.) discloses an enzyme from the soil microorganism *Desmospora sp.* that has psicose-3-epimerase activity. This enzyme is shown to convert D-fructose to D-allulose (also known as D-psicose).

US Patent 8,030,035 and WO2011/040708 disclose an enzyme derived from *Agrobacterium tumefaciens* that has psicose-3-epimerase activity.

Chan et al (Protein Cell. 2012 Feb;3(2):123-31, "Crystal structures of D-psicose 3-epimerase from Clostridium cellulolyticum H10 and its complex ketohexose sugars" also discloses an enzyme that can epimerize D-fructose to yield D-psicose.

There remains a need in the art to discover other genes that express psicose-3-epimerase activity to improve efficiency of converting fructose to allulose in a cost efficient manner. The present invention discloses a novel class of psicose-3-epimases from the betaproteobacteria *Burkholderia* which is a symbiont found the posterior midgut of the bean insect *Riptortus pedestris.* This symbiotic microorganism is thought to be essential for host survival and reproduction and plays pivotal roles in host metabolism by providing essential nutrients, digesting food materials and/ or influencing host plant use, resistance against parasitoids and body color change. Once identified and isolated, an exemplary psicose-3-epimase gene from a *Burkholderia* sp. strain RPE64 was expressed in an *E. coli* production strain and the enzyme was isolated and evaluated in both benchtop and pilot scale production environments. The enzyme was evaluated for commercial allulose production by immobilization of the enzyme on a solid matrix weak base anion exchange resin as described in more detail herein.

### SUMMARY OF THE INVENTION

The present disclosure describes a method of producing allulose comprising, contacting a solution containing fructose with a psicose-3-epimerase enzyme from a *Burkholderia* species having at least 84% sequence identity to SEQ ID NO:1 for a time and under conditions suitable to convert at least a portion of the fructose to allulose. Certain embodiments include a method wherein the psicose-3-epimerase is immobilized on a solid matrix resin and used to convert fructose to allulose, preferably at temperatures between 50° and 70°C. Preferred embodiments include a method wherein the solid matrix is a weak base anion exchange resin.

Exemplary embodiments include a method wherein the solid matrix resin is a phenol formaldehyde based condensate resin functionalized to contain tertiary amine free base groups, exemplified by DUOLITE^{™} A568. Additional exemplary embodiments include a method wherein the solid matrix resin is a methacrylic acid based resin functionalized to contain C2-C6 amine linkages, such as Lifetech^{™} ECR8315, Lifetech^{™} ECR 8415, and SEPABEADS^{™} EC-HA.

Certain embodiments include a method wherein the *Bulkholderia sp.* is selected from the group consisting of *Burkholderia RP64, Candidatus Burkholderia verschuerenii, Burkholderia jiangsuensis, Burkholderia sp. MR1, Burkholderia grimmiae,* and *Candidatus Burkholderia brachyanthoides.*

Preferred embodiments include a method wherein the psicose-3-epimerase enzyme has a polypeptide sequence selected from the group consisting of SEQ ID NO:1, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO:20, SEQ ID NO:21, SEQ ID NO:22, SEQ ID NO:23, SEQ ID NO:24, SEQ ID NO:25, and SEQ ID NO:26.

Additional embodiments of the invention include a method wherein the fructose solution is selected from the group consisting of solubilized crystalline fructose and high fructose com syrup (HFCS). Preferred embodiments include a method of wherein the fructose solution has a dissolved solids content of about 50%w/w, the contacting is done at a pH range of 6.5-7.7 in the presence of magnesium at a concentration of 24-50ppm, and is done with a flow rate of 1-4 volumes of fructose solution per bed volume of the solid matrix resin.

Other embodiments include a method wherein the psicose-3-epimirase enzyme is obtained from a microorganism containing a recombinant nucleic acid vector operably configured to express a nucleic acid sequence encoding the protein having at least 84% sequence identity to SEQ ID NO: 1. Preferred embodiments include a method wherein the microorganism is selected from the group consisting of *E. coli* and *B. subtillis.*

An additional aspect of the invention is a recombinant nucleic acid sequence operably configured to express a nucleic acid sequence encoding a protein having at least 84% sequence identity to SEQ ID NO: 1 in a microorganism.

Another aspect of the invention is a microorganism transformed with the recombinant nucleic acid sequence operably configured to express a nucleic acid sequence encoding a protein having at least 84% sequence identity to SEQ ID NO: 1 in a microorganism. Preferred embodiments include a microorganism selected from the group consisting of *E. coli* and *B. subtillis.*

Additional aspects of the invention include a solid matrix resin containing a psicose-3-epimerase enzyme having at least 84% sequence identity to SEQ ID NO: 1 immobilized thereon. Further aspects include a column containing the solid matrix resin which contains a psicose-3-epimerase enzyme having at least 84% sequence identity to SEQ ID NO:1 immobilized thereon; and, configured to receive an input flow of a solution to flow over the solid matrix resin and permit exit of an output flow of the solution.

### BRIEF DESCRIPTION OF THE DRAWINGS

Figure 1 depicts the bio-conversion of D-fructose to D-allulose.
Figure 2 depicts a vector map of the gene cassette BRPV3 from *Burkholderia sp. RPE64* inserted into the expression vector pET-22b(+).
Figure 3 depicts an evaluation of the psicose-3-epimerase enzyme in a cell free lysate.
Figure 4 depicts results of example 1 in a SDS page gel analyzing different fraction after small scale purification.
Figure 5 depicts results from a large scale expression and purification of psicose-3-epimerase in an SDS page gel analyzing a different fraction after purification as described in Example 2.
Figure 6 depicts the evaluation of the thermostability of the psicose-3-epimerase enzyme as described in Example 3.
Figure 7 depicts the bioconversion of fructose to allulose catalyzed by the pBRPV3 strain at 70°C as described in Example 3.
Figure 8 depicts the enzyme specific activity over the course of pilot scale fermentation as described in Example 6.
Figure 9 depicts a protein sequence of SEQ ID NO:1 which is the psicose-3-epimerase gene from *Burkholderia* RPE64.
Figure 10 depicts a nucleotide sequence of SEQ ID NO:2 which encodes the protein sequence of SEQ ID NO:1.
Figure 11 depicts a nucleotide sequence of the 5' primer used to amplify Seq ID NO: 10 and to append the NdeI restriction site for cloning purposes to construct p-3-13_pET-22b(+). (SEQ ID NO:3)
Figure 12 depicts a nucleotide sequence of the 3' primer used to amplify Seq ID NO: 10 and to append the XhoI restriction site for cloning purposes to construct p-3-13_pET-22b(+) and a stop codon. (SEQ ID NO:4)
Figure 13 depicts a nucleotide sequence of the primer used to amplify the *thrC* gene and to append the DraIII restriction site for cloning purposes to create the E. coli strain BL21(DE3)*thrC-.* (SEQ ID NO:5)
Figure 14 depicts a nucleotide sequence of the primer used to amplify the *thrC* gene and to append the DraI restriction site for cloning purposes to create the E. coli strain BL21(DE3)*thrC-.* (SEQ ID NO:6)

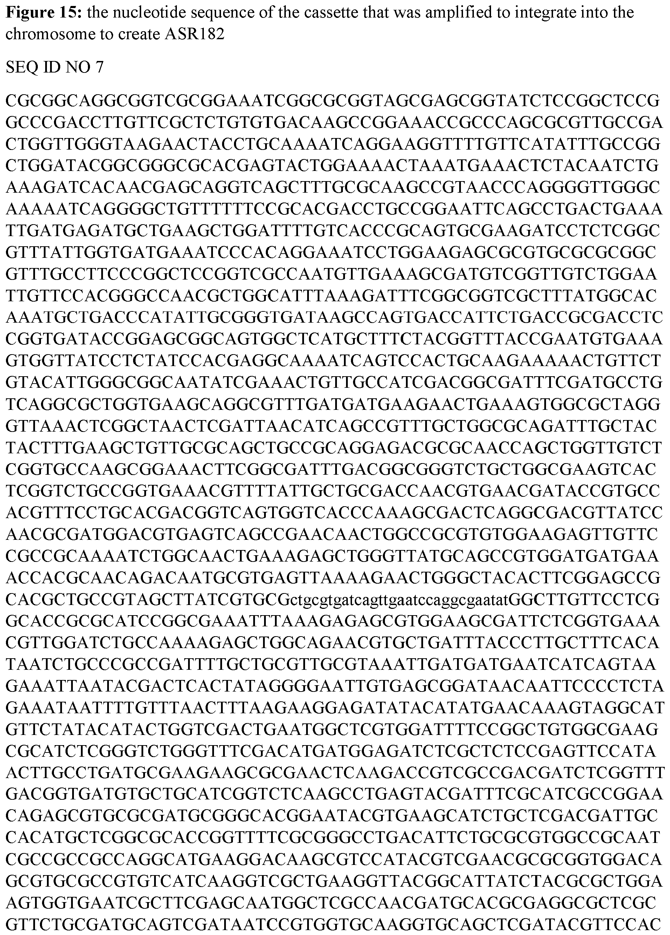

Figure 15 depicts a nucleotide sequence (SEQ ID NO: 7) of the cassette that was amplified to integrate into the chromosome to create ASR182.
Figure 16 depicts SEQ ID NO:8, the nucleotide sequence of the 5' primer used to amplify SEQ ID NO:7.
Figure 17 depicts SEQ ID NO:9, the nucleotide sequence of the 3' primer used to amplify SEQ ID NO:7.
Figure 18 depicts production of psicose-3-epimerase by ASR180 over the course of the fermentation as described in Example 6.

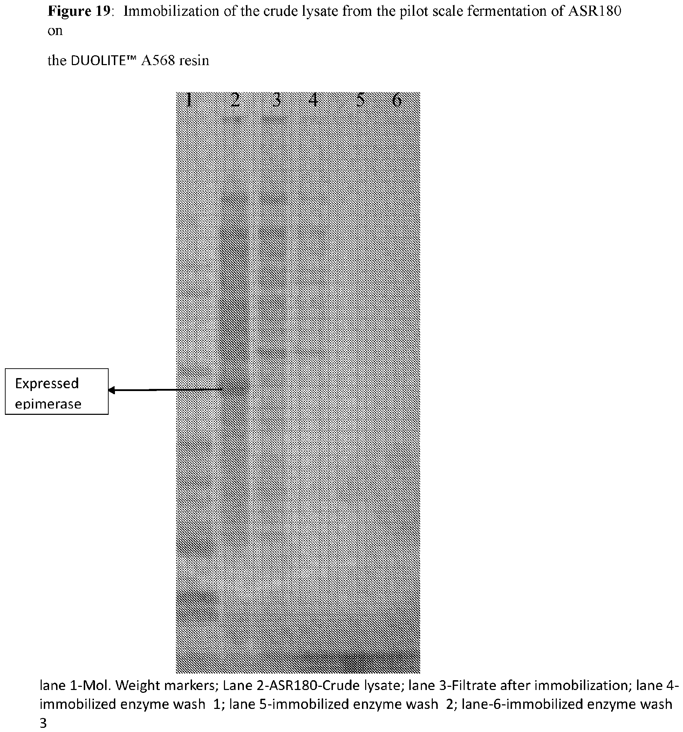

Figure 19 depicts immobilization of a crude lysate from the pilot scale fermentation of ASR180 on the DUOLITE^{™} resin.
Figure 20 depicts conversion of fructose to allulose using psicose-3-epimerase from *Burkholderia sp.* REP4 immobilized on DUOLITE^{™} resin as described in Example 7.
Figure 21 depicts conversion of fructose to allulose using psicose-3-epimerase from *Burkholderia Sp. REP4* immobilized on Lifetech^{™} ECR8415 resin as seen in Example 8.
Figure 22 depicts results from the enzyme assay as described in Example 9.
Figure 23 depicts a vector plasmid p-3-13_pET-22b(+)*thrC*. This was used to create the production strain ASR180.
Figure 24 depicts the nucleotide sequence (SEQ ID NO: 10) that encodes the protein sequences according to SEQ ID NO: 1 with the exception of an additional NdeI restriction site at the 5' end and an XhoI restriction site at the 3' end. The restriction sites are highlighted in gray.
Figure 25 depicts SEQ ID NO: 11 which is the protein sequence of tagatose-3-epimerase enzyme from *Pseudomonas cichorii.*
Figure 26 depicts the protein sequence for *a Burkholderia* psicose 3-epimerase according to SEQ ID NO: 12.
Figure 27 depicts the protein sequence for a *Burkholderia* psicose 3-epimerase according to SEQ ID NO: 13.

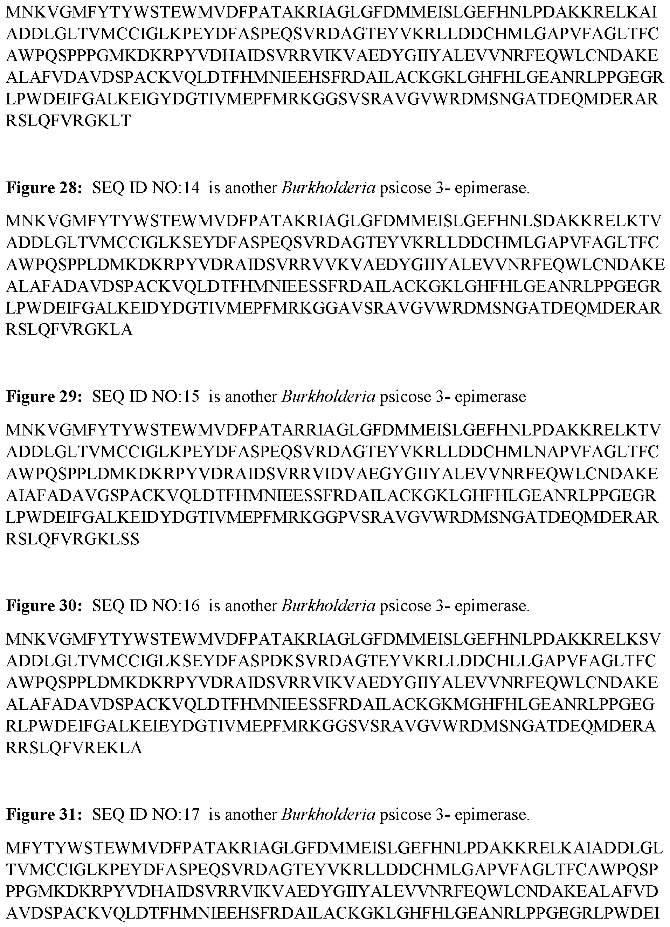

Figure 28 depicts the protein sequence for a *Burkholderia* psicose 3-epimerase according to SEQ ID NO: 14.
Figure 29 depicts the protein sequence for a *Burkholderia* psicose 3-epimerase according to SEQ ID NO: 15.
Figure 30 depicts the protein sequence for *a Burkholderia* psicose 3-epimerase according to SEQ ID NO: 16.
Figure 31 depicts the protein sequence for a *Burkholderia* psicose 3-epimerase according to SEQ ID NO: 17.

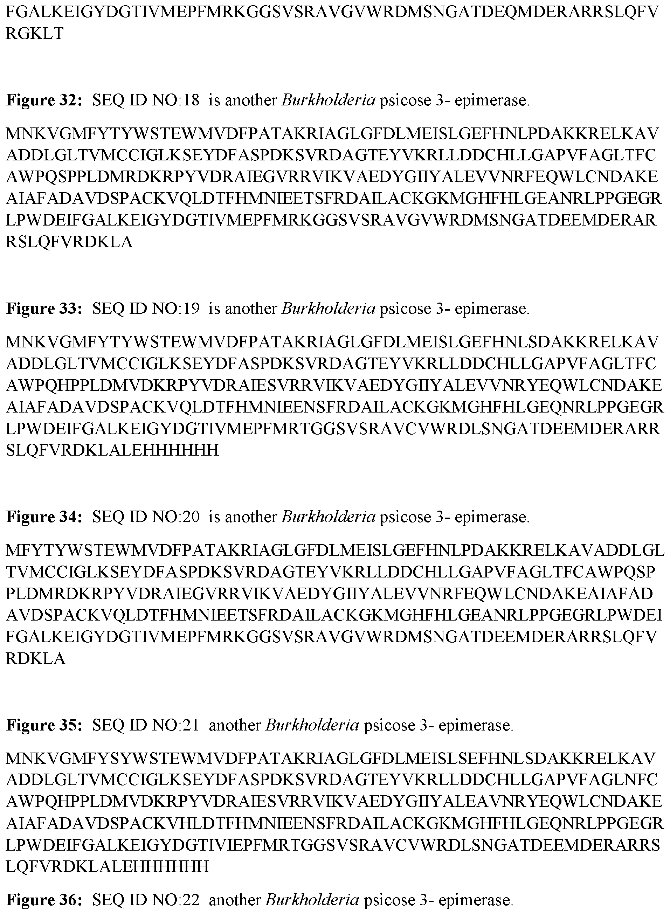

Figure 32 depicts the protein sequence for a *Burkholderia* psicose 3-epimerase according to SEQ ID NO: 18.
Figure 33 depicts the protein sequence for a *Burkholderia* psicose 3-epimerase according to SEQ ID NO: 19.
Figure 34 depicts the protein sequence for a *Burkholderia* psicose 3-epimerase according to SEQ ID NO:20.
Figure 35 depicts the protein sequence for a *Burkholderia* psicose 3-epimerase according to SEQ ID NO:21.
Figure 36 depicts the protein sequence for *a Burkholderia* psicose 3-epimerase according to SEQ ID NO:22.

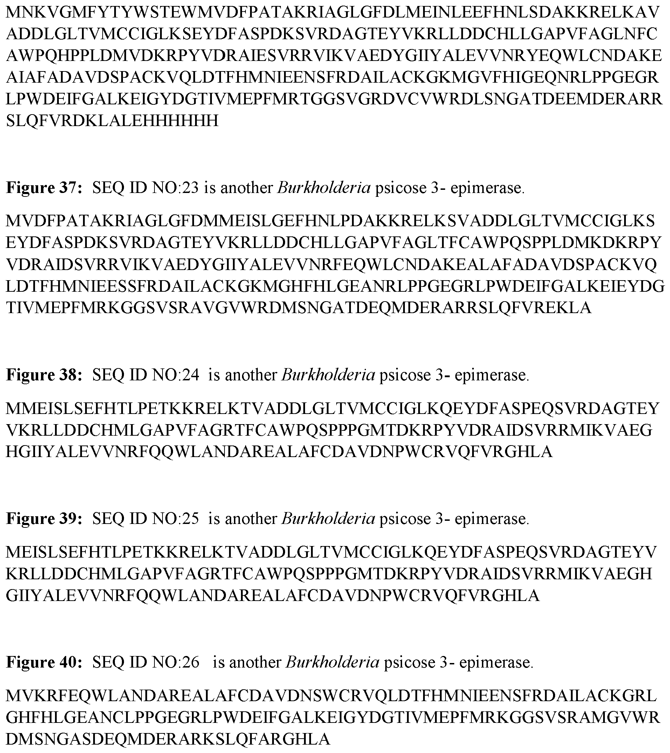

Figure 37 depicts the protein sequence for a *Burkholderia* psicose 3-epimerase according to SEQ ID NO:23.
Figure 38 depicts the protein sequence for *a Burkholderia* psicose 3-epimerase according to SEQ ID NO:24.
Figure 39 depicts the protein sequence for *a Burkholderia* psicose 3-epimerase according to SEQ ID NO:25.
Figure 40 depicts the protein sequence for a *Burkholderia* psicose 3-epimerase according to SEQ ID NO:26.

### SEQUENCE LISTING

SEQ ID NO:1 is the psicose-3-epimerase gene from *Burkholderia* RPE64.
SEQ ID NO:2 is the nucleotide sequence which encodes the protein sequence of SEQ ID NO:1.
SEQ ID NO:3 is the nucleotide sequence of the 5' primer used to amplify Seq ID NO: 10 and to append the NdeI restriction site for cloning purposes to construct p-3-13_pET-22b(+).
SEQ ID NO:4 is the nucleotide sequence of the 3' primer used to amplify Seq ID NO: 10 and to append the XhoI restriction site for cloning purposes to construct p-3-13_pET-22b(+) and a stop codon.
SEQ ID NO: 5 is the nucleotide sequence of the primer used to amplify the *thrC* gene and to append the DraIII restriction site for cloning purposes to create the E. coli strain BL21(DE3)*thrC*-.
SEQ ID NO:6 is the nucleotide sequence of the primer used to amplify the *thrC* gene and to append the DraI restriction site for cloning purposes to create the E. coli strain BL21(DE3)*thrC*-.
SEQ ID NO:7 is the nucleotide sequence of the cassette that was amplified to integrate into the chromosome to create ASR182.
SEQ ID NO:8 is the nucleotide sequence of the 5' primer used to amplify SEQ ID NO:7.
SEQ ID NO:9 is the nucleotide sequence of the 3' primer used to amplify SEQ ID NO:7.
SEQ ID NO: 10 is the nucleotide sequence that encodes the protein sequences according to SEQ ID NO: 1 with the exception of an additional NdeI restriction site at the 5' end and an XhoI restriction site at the 3' end. The restriction sites are highlighted in gray.
SEQ ID NO: 11 is the protein sequence of tagatose-3-epimerase enzyme from *Pseudomonas cichorii.*
SEQ ID NO: 12 is a protein sequence for another *Burkholderia* psicose 3-epimerase.
SEQ ID NO: 13 is a protein sequence for another *Burkholderia* psicose 3-epimerase.
SEQ ID NO: 14 is a protein sequence for another *Burkholderia* psicose 3-epimerase.
SEQ ID NO: 15 is a protein sequence for another *Burkholderia* psicose 3-epimerase.
SEQ ID NO: 16 is a protein sequence for another *Burkholderia* psicose 3-epimerase.
SEQ ID NO: 17 is a protein sequence for another *Burkholderia* psicose 3-epimerase.
SEQ ID NO: 18 is a protein sequence for another *Burkholderia* psicose 3-epimerase.
SEQ ID NO: 19 is a protein sequence for another *Burkholderia* psicose 3-epimerase.
SEQ ID NO:20 is a protein sequence for another *Burkholderia* psicose 3-epimerase.
SEQ ID NO:21 is a protein sequence for another *Burkholderia* psicose 3-epimerase.
SEQ ID NO:22 is a protein sequence for another *Burkholderia* psicose 3-epimerase.
SEQ ID NO:23 is a protein sequence for another *Burkholderia* psicose 3-epimerase.
SEQ ID NO:24 is a protein sequence for another *Burkholderia* psicose 3-epimerase.
SEQ **ID** NO:25 is a protein sequence for another *Burkholderia* psicose 3-epimerase.
SEQ **ID** NO:26 is a protein sequence for another *Burkholderia* psicose 3-epimerase.

### DETAILED DESCRIPTION OF THE INVENTION

The following description and foregoing background makes citations to certain references that may aid one of ordinary skill in the art to understand the present invention and that may provide material, information, techniques, proteins, vectors and nucleotide sequences that may assist one of ordinary skill in the art to make and use aspects of the present invention in its fullest scope. Accordingly, each cited reference is incorporated into this application as if originally filed herewith to the extent the teaching of the cited references do not conflict with the teaching of the present application, in which case the teaching of this application shall be deemed to control over the conflicting teaching of art incorporated herein by reference.

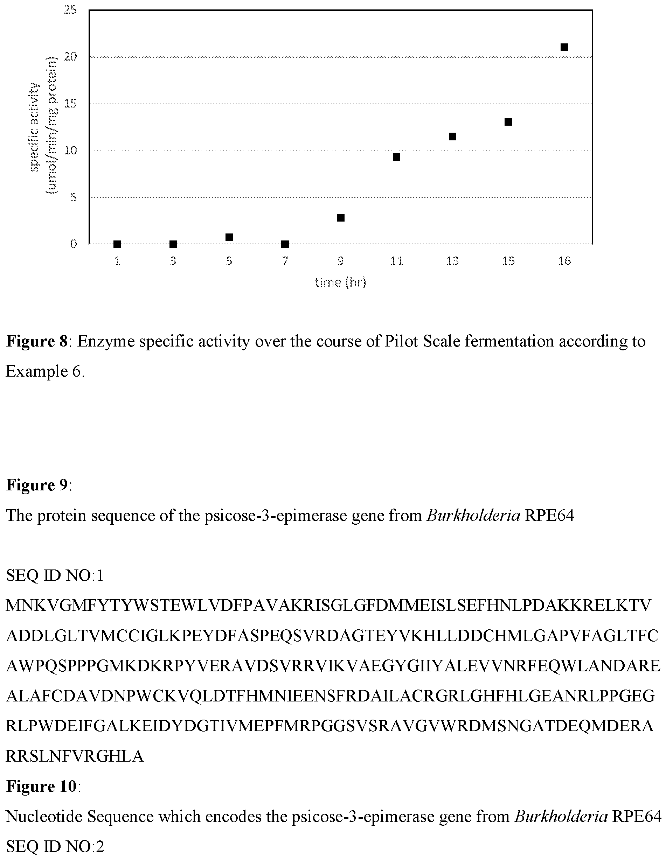

One aspect of the present invention is the discovery of a class of psicose-3-epimerase enzymes encoded by genes from the betaproteobacterial genus *Burkholderia,* which is exemplified herein by SEQ ID NO: 1, which is the protein sequence of a psicose-3-epimerase from a *Burkholderia sp* strain RPE 64 which is a symbiont found in the posterior midgut of the bean insect *Riptortus pedestris.* Other examples of psicose-3-epimerase genes from this class are shown in Table 1, all of which share at least 84% amino acid sequence identity to SEQ ID NO:1 and all of which substantially differ from other psicose-3-epimerases disclosed for use in converting fructose to allulose, such as those from *Clostridium sp., Agrobacterium tumefaciens*, *and Desmospora sp.* that have previously been described in the art. SEQ ID NO: 1 only has 42% identity to the *Desmospora sp* polypeptide sequence disclosed by Woodyer *et al.* Likewise, the *Clostridium cellulolyticum* sequence described by Chan *et al.* has 42% identity to SEQ ID NO:1 and the *Agrobacterium tumefaciens* polypeptide described by US Patent 8,030,035 and WO2011/040708 only showed 41% identity to SEQ ID NO 1. An exception to this rule is the epimerase from *Pseudomonas cichorii,* (SEQ ID NO: 11) shown in Figure 10, which has 88% sequence identity to SEQ ID NO: 1 but which is excluded from the present invention.

**Table 1**

| Exemplary species of *Burkholderia* psicose-3-epimerase | | |
|---|---|---|
| **SEQ ID NO:** | ***Burkholderia* species:** | **% Identity** |
| 12 | Candidatus Burkholderia verschuerenii | 94 |
| 13 | Burkholderia jiangsuensis | 92 |
| 14 | Burkholderia sp. | 91 |
| 15 | Burkholderia sp. | 91 |
| 16 | Burkholderia sp. MR1 | 90 |
| 17 | Burkholderia jiangsuensis | 92 |
| 18 | Burkholderia grimmiae | 88 |
| 19 | unknown | 87 |
| 20 | Burkholderia grimmiae | 88 |
| 21 | unknown | 85 |
| 22 | unknown | 84 |
| 23 | Burkholderia sp. MR1 | 89 |
| 24 | Candidatus Burkholderia brachyanthoides | 89 |
| 25 | Candidatus Burkholderia brachyanthoides] | 88 |
| 26 | Candidatus Burkholderia calva] | 91 |

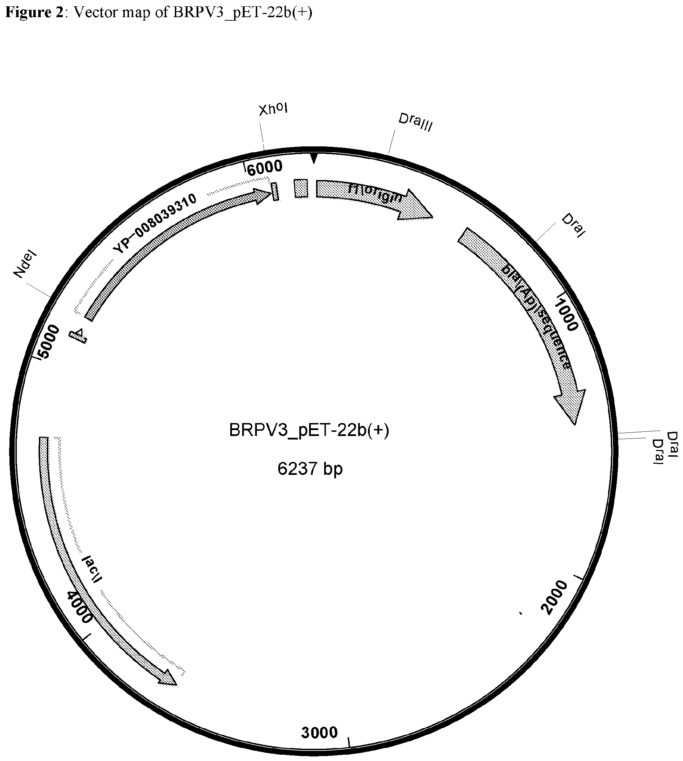

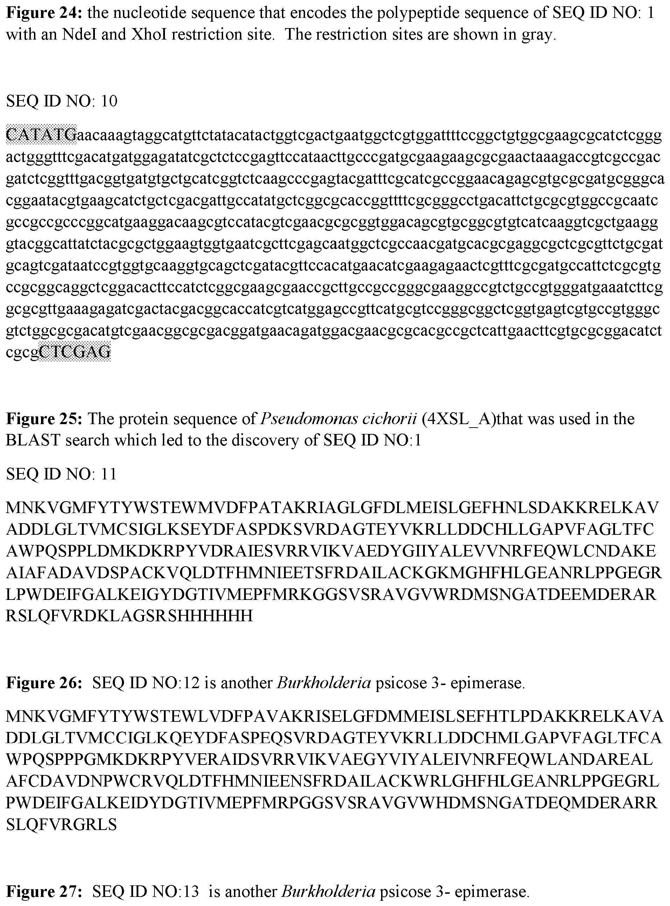

SEQ ID NO: 1: was identified in a database by the National Center for Biotechnology Information (NCBI) as accession number YP_008039310 and was labeled as being a xylose isomerase gene. Prior to the present invention, it was not known that this enzyme has a psicose-3-epimerase activity capable of isomerizing fructose to allulose. In order to determine that the exemplary enzyme of the present invention displayed epimerase activity, the present inventors had the gene encoding the protein identified by accession number YP_008039310 chemically synthesized by GenScript (Biscataway, NJ). The synthetic DNA was created to include NdeI and XhoI restriction sites at the 5' and 3' ends of the polynucleotide in order to ensure simple cloning. The sequence of this synthesized polynucleotide is according to SEQ ID NO: 10 as shown in Figure 24 and is referred to herein as the BRPV3 gene cassette. The BRPV3 cassette was designed with the NdeI and XhoI restriction sites and was cloned into a NdeI/XhoI digested expression vector pET-22b+ obtained from Novagen, Inc. (Madison, WI) so the N terminus would be in frame with a polyhistidine tag sequence contained within the vector. The resulting expression vector is herein referred to as BRPV3_pET22b(+), a map of which is provided in Figure 2.

The expression vector BRPV3_pET22b(+) was transformed into the *E*. *coli* strain BL21(DE3) (Studier, FW and BA Moffatt. Use of bacteriophage T7 RNA polymerase was used to direct selective high-level expression of cloned genes. J Mol Biol. 1986 May 5; 189(1):113-130). The strain that resulted from this transformation is herein referred to as BL21(DE3)/BRPV3.

Single colonies were selected and incubated overnight (30°C, 250 rpm, 16 h) in 5 ml LB broth (Miller) (BD Biosciences, Inc.) with 100 µg/µL ampicillin (Sigma-Aldrich Corp.) to maintain the plasmids. After 16 h, 2 ml of each culture was diluted 1:100 in a baffled shake flask containing 200 mL LB (100 µg/µL ampicillin) and grown until OD_{600 nm} reached approximately 0.5 (30°C, 250 rpm). Then the cultures were induced with a final concentration of 0.5 mM Isopropyl β-D-1-thiogalactopyranoside (IPTG from Sigma-Aldrich Corp.) and incubated again for 16 h. Cells were harvested by 5 minute centrifugation at 5,000 rpm, 4°C. The supernatant was discarded and cell pellets were frozen at -80°C.

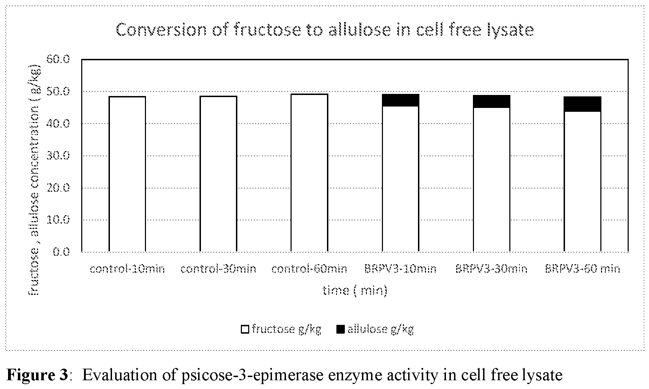

The frozen cells were thawed on ice, lysed, and centrifuged. The supernatant was used to evaluate if the expressed gene could catalyze epimerization of fructose to allulose, which confirmed that SEQ ID NO: 1 in fact encoded an enzyme with the epimerase enzyme activity. Results of this experiment can be seen in Figure 3.

While there was clear demonstration of the plasmid BRPV3_pET-22(b)+ expressing the encoded enzyme in the *E. coli* strain BL21(DE3)/BRPV3, a strain free of antibiotic markers was ultimately desired. In order to accomplish this, the *E. coli* strain BL21(DE3)*thrC-* was created, which is auxotrophic for threonine
biosynthesis. BL21(DE3)*thrC*- was generated by the integration of an antibiotic cassette deleting *thrC* and excision of the antibiotic cassette by the following steps. The *thrC* gene is already disrupted in an *E. coli* K-12 derivative in the Keio collection (Baba, T, T Ara, M Hasegawa, Y Takai, Y Okumura, M Baba, KA Datsenko, M Tomita, BL Wanner, and H Mori. Construction of Escherichia coli K-12 in-frame, single-gene knockout mutants: the Keio collection. Mol Syst Biol. 2006; 2:2006.0008*).* In this *E. coli* K-12 derivative, a kanamycin cassette has replaced the native *thrC* gene and is flanked by DNA sequences recognized by the FLP recombinase, an enzyme capable of excising the cassette. A P1 phage lysate generated from this *thrC*::km^{R} strain was used to transduce kanamycin resistance into BL21 (DE3). The transductants were transformed with a temperature sensitive ampicillin resistant plasmid pCP20 (Cherepanov, PP and W Wackernagel. Gene disruption in Escherichia coli: TcR and KmR cassettes with the option of Flp-catalyzed excision of the antibiotic-resistance determinant. Gene 158:9-1) to transiently introduce the FLP recombinase and isolates that were sensitive to kanamycin and ampicillin were confirmed as being auxotrophic for threonine biosynthesis.

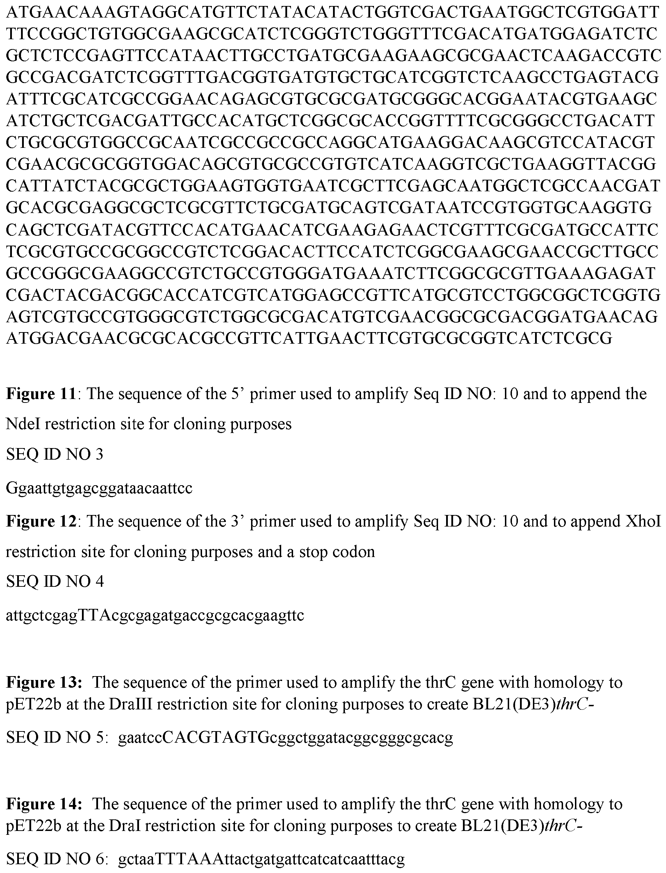

In parallel, the plasmid BRPV3_pET-22(b)+ was used as the template in a PCR reaction where the primers shown in Figures 10 and 11 (SEQ ID NO:2 and 3, respectively) were used to amplify the polynucleotide sequence that expresses the epimerase activity. The amplified PCR product and pET-22(b)+ were digested with the restriction enzymes NdeI and XhoI and ligated together to create the plasmid p-3-13_ pET-22(b)+. The 3' primer (SEQ ID NO: 3) contains a stop codon upstream of the XhoI restriction site so, once ligated, the His tag of the pET-22(b)+ expression vector is no longer in frame.

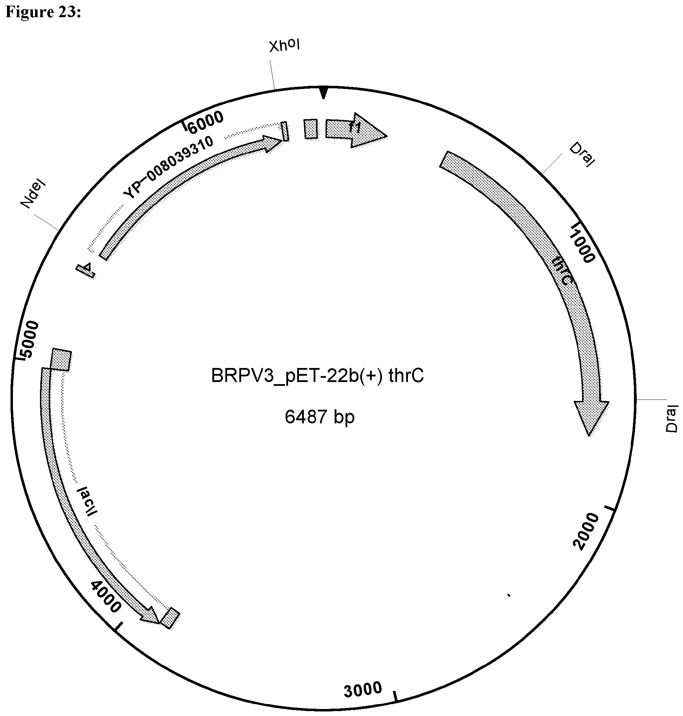

The plasmid p-3-13_pET-22b (+) was digested with DraI and DraIII NEB restriction enzymes to remove all ampicillin resistance-encoding DNA. The *thrC* gene of *E. coli* K-12 was amplified with primers SEQ ID NO:4 and NO:5 (as seen in Figures 12 and 13) that appended extended homology to pET22b at the DraI and DraIII cut sites. The PCR and digested p-3-13_pET-22(b)+ plasmid were assembled by means of a Gibson Assembly Master Mix (NEB). The resulting plasmid, p-3-13_pET-22(b)+thrC, was transformed into BL21(DE3)*thrC-* and the colonies that appeared on M9 minimal media constitute the strain designated herein as ASR180. ASR180 is an *E*. *coli* strain containing the antibiotic resistant marker free vector as indicated in Figure 23.

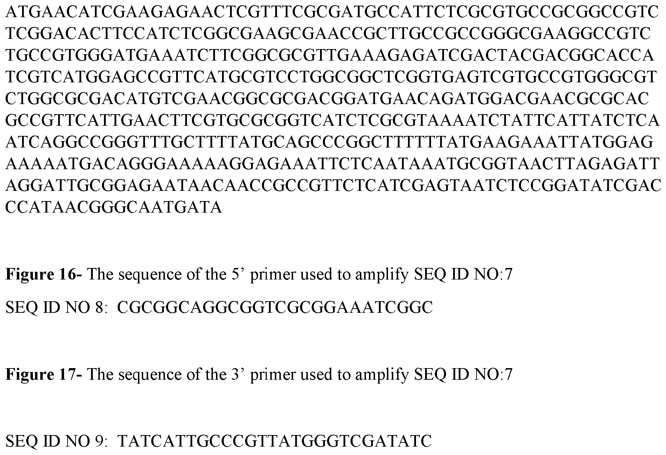

In order to construct ASR182, which is an *E. coli* strain with a copy of the psicose-3-epimerase gene from *Burkholderia* RPE64 integrated into the chromosome, a synthetic nucleotide cassette was made by Integrated DNA Technologies (Coralville, IA) according to SEQ ID NO: 7. The cassette functionally encodes a small piece of homologous DNA upstream of the *thrC* gene, the *thrC* gene, a T7 promoter, a sequence encoding the enzyme according to SEQ ID NO:1 and a small piece of homologous DNA downstream of *thrC.* This cassette was amplified with Go Taq (Promega, Inc., Fitchburg, WI) using IDT primers according to SEQ ID NO: 8 and 9 (Figures 16 and 17, respectively). The amplified cassette and the plasmid pKD46 (Datsenko, KA and BL Wanner. One-step inactivation of chromosomal genes in Escherichia coli K-12 using PCR products. Proc Natl Acad Sci USA. 2000 Jun 6; 97(12): 6640-6645*)* was introduced into strain BL21 (DE3)*thrC-,* previously described herein. As described more fully in Datsenko et al., the plasmid pKD46 has a recombination mechanism capable of integrating the amplified cassette into the chromosome of BL21(DE3)*thrC*-. The cassette was integrated into the chromosome of BL21(DE3)*thrC-* by selecting for prototrophy on M9 minimal plates. The strain was cured of pKD46 by passage at 37°C. Prototrophic isolates sensitive to ampicillin and expressing the desired enzymatic activity were characterized.

While the enzyme exemplified herein was obtained by expression of a vector encoding the enzyme in *E. coli,* one of ordinary skill in the art can overexpress any of the psicose-3-epimerases enzymes in any suitable microorganism, for example *B*. *subtilis* is another preferred bacteria for overexpressing enzymes.

Once the strains ASR180 and ASR182 were constructed, small benchtop fermentations and large pilot scale fermentations were done as shown in the following examples. Isolation of the psicose-3-epimerase enzyme that was produced from these fermentations was done by immobilization onto solid matrix resins described in further detail in the examples below.

### EXAMPLES

The following examples are offered for purposes of illustration and are not intended to limit the scope of the invention.

**Example 1:** Small scale production of BL21DE3/BRPV3: After construction of the BL21DE3/BRPV3 strain, cultivation was done by first obtaining single colonies. Selected single colonies were inoculated into 15 mL of LB medium containing ampicillin (100 ug/mL) held in 50 mL flask. Cultures were incubated with shaking at 250 rpm at 30°C on New Brunswick Scientific G25 Gyratory shakers. A 1% inoculum derived from overnight stage I cultures was used to initiate fresh LB cultures (500 mL) with antibiotics in a 2.7 L Fembach flask. The culture was incubated at 30°C for 16h with shaking at 250 rpm on a gyratory shaker. When the optical density was 0.9, the expression of protein was induced with IPTG to a final concentration of 0.5 mM. *E. coli* cells were harvested by centrifugation at 5,474 x g for 6 min at 4°C. The cells were lysed and the psicose-3-epimerase enzyme was purified using a Ni-NTA column. The eluted protein was dialyzed with tris buffer and evaluated for enzyme activity according to the procedure as described herein in Example 3.

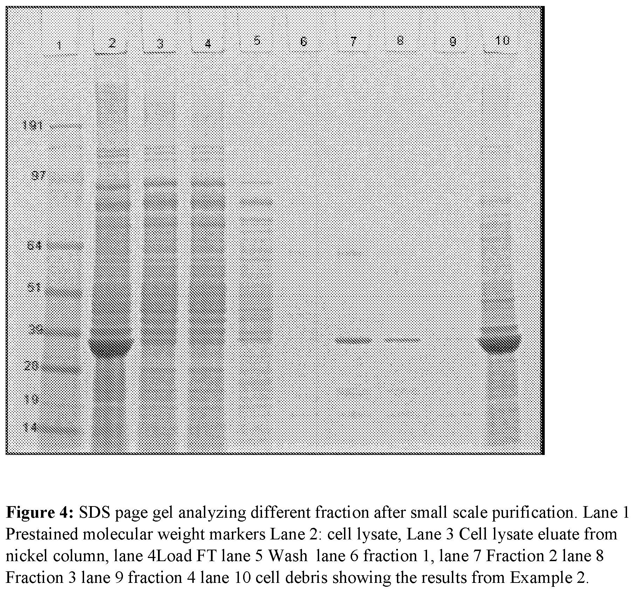

The results of this evaluation can be seen in Figure 4, which is a SDS page gel analyzing different fraction after small scale purification. Lane 1 is a Pre-stained molecular weight markers, Lane 2 is cell lysate, Lane 3 is cell lysate eluate from the nickel column, Lane 4 is Load FT, Lane 5 is Wash, Lane 6 is fraction 1, Lane 7 is Fraction 2, Lane 8 is Fraction 3, Lane 9 is fraction 4, and Lane 10 is cell debris.

**Example 2:** Large scale purification of psicose-3-epimerase from of BL21DE3/BRPV3

BL21(DE3)/BRPV3 was cultured in a Biostat ^{®} C-plus (Sartorius) fermenter. Seed cultures were initiated by inoculating 50 µl of frozen glycerol stock in a 250 mL flask containing 60 mL of LB Lenox media with ampicillin (100 ug/mL).

The flask was shaken at 30°C, 250 rpm for 16 hrs. The overnight culture were transferred to Biostat ^{®} C-plus (Sartorius) fermenters containing 12 L of LB Lennox media with ampicillin (100 ug/mL). The fermenter was maintained at 30°C, 30 % dissolved oxygen and at pH 6.8 ± 0.1. pH was adjusted with either 2N HCL or 5N NaOH. When the culture attained an OD600 of 1, the culture was induced with β-isopropyl thiogalactoside (IPTG) (final conc 0.5 mM). After the culture was grown overnight, the fermentation was stopped and cells were harvested by centrifugation. The cells were stored at -80°C until further use.

The frozen cells were thawed and suspended in 100 mL lysis buffer (15 mM Tris Cl, 10 mM imidiazole, 300 mM NaCl, 2mM MnCl₂, ph7.5). The cell were lysed using a microfluidizer (18,000 psi). The cell lysate was centrifuged at 15,000 g to separate cell debris. The supernatant was used for further purification.

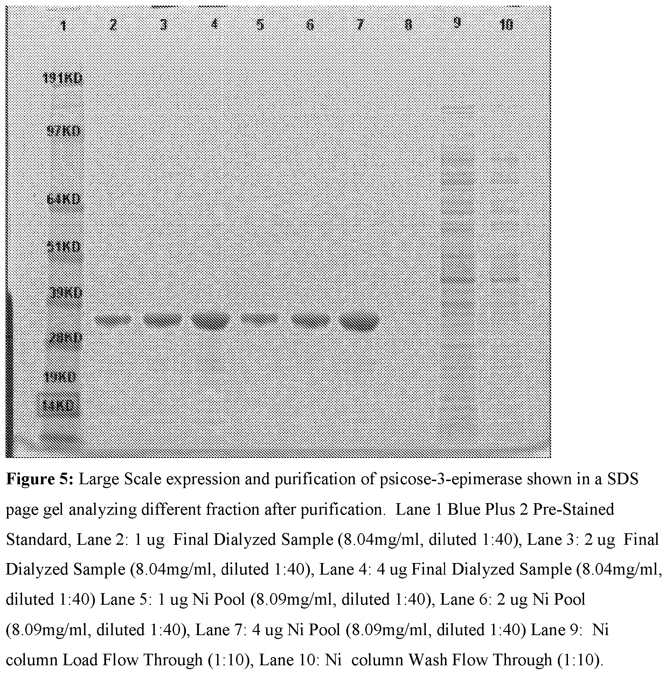

The active enzyme was purified using AKTA explorer FPLC system equipped with 40 mL of Ni-NTA column. The column was equilibrated with the lysis buffer and the supernatant was loaded onto the column at 6 ml/min. After all the cell lysate was loaded, the column was washed with 5 bed volumes of wash buffer (15 mM Tris. Cl, 30 mM imidiazole, 300 mM NaCl, 2mM MnCl₂, pH 7.5) to separate out all the other undesired proteins. The Ni-NTA column was then further washed with elution buffer (15 mM Tris. Cl, 250 mM imidiazole, 300 mM NaCl, 2mM MnCl₂, pH 7.5) to elute the His tagged enzyme from the column. The fraction containing active enzyme was pooled, concentrated and dialyzed with 15 mM TrisCl, 2mM MnCl₂, 50% glycerol pH 7.5. The stabilized enzyme was stored at 4°C. The purified enzyme was stable for > 2 years when stored under these conditions. The enzyme containing fractions were run on an SDS page gel and the results can be seen in Figure 5. The enzyme activity was evaluated according the methods described herein in Example 3.

**Example 3:** Purified psicose-3-epimerase enzyme from the fermentation as described in Example 1 and 2 was evaluated using an epimerase assay performed at 50°C for 5 min in 50 mM TrisCl buffer (pH 7.5) containing 200 mM fructose and 40 ug of purified enzyme. The reaction was stopped by heating at 105 °C for 10 min. One unit of D-psicose 3-epimerase activity was defined as the amount of the enzyme required to produce 1 umol of psicose per min at pH 7.5 and 50°C.

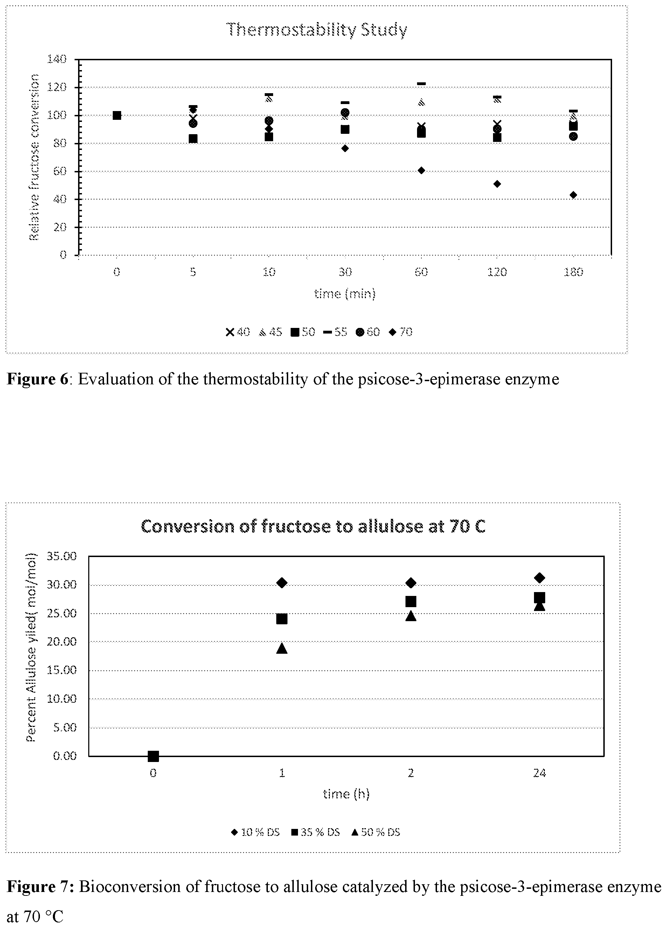

Purified enzyme was diluted 10-fold into 50 mM tris buffer containing 2 mM MnCl₂, pH 7.5. This solution was incubated at different temperature (40-70 °C). At appropriate time after incubation at the desired temperature, an aliquot of enzyme sample (50 uL) was withdrawn and evaluated for residual activity. The purified enzyme showed a similar level of enzyme activity at all temperatures tested. It was observed that the enzyme was catalytically active after incubating at 70 °C for 3 hrs. These results can be seen in Figure 6. The bioconversion of fructose to allulose at 70 °C can also be seen in Figure 7.

**Example 4:** Immobilization of psicose-3-epimerase on solid matrix resins. The cell lysate from BL21(DE3)/BRPV3 as grown under the condition disclosed in Example 1 was screened for epimerase activity on several immobilization support resins. The results comparing the resins can be seen in Table 2 showing the activity of the present enzyme measured by GC analysis, which indicate that weak base solid matrix resins are the best support for immobilizing this enzyme. Table 2 further suggests that strong anion exchange resins, such as Lifetech^{™} ECR1504, Lifetech^{™} ECR1640, and Lifetech^{™} ECR1604, are not most suitable support resins for immobilizing the enzyme of the present invention.

Where there are two numbers in a row of Table 2, the bolded bottom number indicates the enzyme activity as measured by HPLC. Most importantly, the last column indicates that the resins ECR8315F/M, ECR8415F/M and DUOLITE^{™} A568 show a very low % of activity left in the supernatant as it passes through the column, indicating a high enzyme loading capacity of these resins. ECR8315 F/M and ECR8415 F/M (Lifetech^{™} ) and DUOLITE^{™} A568 were identified as optimal immobilization support for this enzyme. SEPABEADS^{™} EC-HA which is also a methacrylic based amino modified resin (similar to ECR8415) will also work for immobilization. Based on this, it can be hypothesized, this psicose-3-epimerase has a high affinity for amino modified resin and shows high activity of the psicose-3-epimerase on these resins once immobilized.

**Table 2:**

| | **Carrier #** | **% water** | **g wet** | **U/g** | **U/g dry** | **% rec** | **% activity in supernatant** |
|---|---|---|---|---|---|---|---|
| 1 | Lifetech^{™} ECR8315F | 78 | 1.13 | 300 | 1350 | 67 | < 1 |
| 2 | Lifetech^{™} ECR8415F | 78 | 1.13 | 408 | 1834 | 92 | <<1 |
| | | | | **363** | **1640** | **82** | |
| 3 | SEPABEADS^{™} EC- | 63 | 0.68 | 700 | 1900 | 95 | 14 |
| | HA | | | **515** | **1392** | **70** | |
| 4 | Lifetech^{™} ECR1504 | 55 | 0.56 | 115 | 256 | 13 | 72 |
| 5 | Lifetech^{™} ECR1640 | 72 | 0.89 | 103 | 370 | 18 | 79 |
| 6 | Lifetech^{™} ECR1604 | 62 | 0.66 | 109 | 286 | 14 | 70 |
| 7 | DUOLITE^{™} A568 | 66 | 0.74 | 636 | 1908 | 95 | 15 |
| | | | | **463** | **1392** | **70** | |

### Example 5: Benchtop Epimerase production

Psicose-3-epimerase was produced in the lab at a scale of 4- 5 liters. A batch process was designed using *E. coli* strain ASR180. This experiment was done with AS182 also, but for exemplary purposes, data and results from AS180 are shown herein. M9 media was used in the flask: 5.0 g/L Dextrose; 12.8 g/L Na2HPO4.7H2O; 5.0 g/L KH2PO4; 1.0 g/L NH4Cl; 0.5 g/L NaCl; 0.3 g/L MgSO4; 0.03 g/L CaCl2; 2.6 g/L (NH4)2SO4; 50 mg/L FeCl3·6 H2O; and 1.8 mg/L ZnSO4·7 H2O. Thiamine was aseptically added at a concentration of 0.05 g/L. 1.5 to 1.8 ml inoculum from a frozen vial was used to inoculate one 50 ml shake flask. Flasks were incubated at 37 °C and 250 RPM for 8 to 9 hours or until an OD660 of 3.5-5.0 units was reached.

When OD660 reached 3.0 to 5.0 units in the shake flask, the culture was transferred to a seed fermenter at a 1.0% ratio. M9 media was used in the seed fermenter: 12.8 g/L Na2HPO4.7H2O; 5.0 g/L KH2PO4; 1.0 g/L NH4Cl; 0.5 g/L NaCl; 0.3 g/L MgSO4; 0.03 g/L CaCl2; 2.6 g/L (NH4)2SO4; 50 mg/L FeCl3·6 H2O; 1.8 mg/L ZnSO4·7 H2O; 0.0 to 1.5 g/L citric acid; and 0.1 ml/L Antifoam. Dextrose and thiamine were aseptically added at a concentration of 5.0 g/L and 0.05 g/L, respectively. Seed tank conditions at EFT=0: temperature was 37 °C, pH was 7.0, agitator was 400 RPM, and airflow was 1.5 vvm. During incubation, pH was controlled with 21% aqueous NH4OH at set point of 7.00, dissolved oxygen was controlled at 35% using agitation (max 1000 RPM), and dextrose concentration was maintained between 2 and 10 g/L. The culture incubated for 12 to 14 hours or until OD660 was 25 to 40 units.

When OD660 reached 25 to 40 units in the seed fermenter, the culture was transferred to a production fermenter at a 2.5% ratio. Media was used in the production fermenter: 2.00 g/L (NH4)2SO4; 8.00 g/L K2HPO4; 2.00 g/L NaCl; 1.00 g/L Na3Citrate.2H2O; 1.00 g/L MgSO4.7H2O; 0.03 g/L CaCl2.2H2O; 0.05 g/L FeSO4.7H2O; 0.0 to 1.5 g/L citric acid; 0.01 ml/L Antifoam; and 0.40 ml/L Neidhardt micronutrients solution. The Neidhardt micronutrients solution consisted of: 0.18 g/L (NH4)6(MO7)24.4H2O; 1.24 g/L H3BO3; 0.36 g/L CoCl2.6H2O; 0.12 g/L CuSO4.5H2O; 0.80 g/L MnCl2.4H2O; and 0.14 g/L ZnSO4.7H2O. Dextrose and thiamine were aseptically added at a concentration of 5.0 g/L and 0.05 g/L, respectively. Production tank conditions at EFT=0: temperature was 37 °C, pH was 7.0, agitator was 400 RPM, and airflow was 1.5 vvm. During the growth phase, pH was controlled with 28% aqueous NH4OH at set point of 7.00, dissolved oxygen was controlled at 35% using agitation (max 1000 RPM), and dextrose concentration was maintained between 2 and 10 g/L. The growth phase lasted until OD660 was 25 to 30 units.

Once the OD 660 reached 25 to 30 units in the production fermenter, the induction phase began. A bolus of 2.00 g/L to 10 g/L lactose or 0.8 mMol/L IPTG and 0.6 to 1.80 g/L yeast extract was added and the temperature was linearly ramped down from 37°C to 30°C over the course of 1 hour. Production tank conditions after induction: temperature was 30°C, pH was 7.0, agitator was 400 to 1000 RPM, and airflow was 1.5 vvm. During the induction phase, pH was controlled with 21% aqueous NH4OH at set point of 7.00 and dextrose concentration was maintained less than 1.0 g/L

### Example 6: Epimerase production at pilot scale

A fed-batch protocol was developed to increase the production of psicose-3-epimerase. *E. coli* strain ASR180 was used. This experiment was done with AS182 also, but for exemplary purposes, data and results from AS180 will be shown herein. M9 media was used in the flask: 5.0 g/L Dextrose; 12.8 g/L Na2HPO4.7H2O; 5.0 g/L KH2PO4; 1.0 g/L NH4Cl; 0.5 g/L NaCl; 0.3 g/L MgSO4; 0.03 g/L CaCl2; 2.6 g/L (NH4)2SO4; 50 mg/L FeCl3·6 H2O; and 1.8 mg/L ZnSO4·7 H2O. Thiamine was aseptically added at a concentration of 0.05 g/L. 1.5 to 1.8 ml inoculum from a frozen vial was used to inoculate two 2L flasks with 250 ml of media. Flasks were incubated at 37°C and 250 RPM for 12 to 14 hours or until an OD660 of 3.5-5.0 units was reached.

When OD660 reached 3.0 to 5.0 units in the shake flask, the culture was transferred to a seed fermenter at a 1.0% ratio. M9 media was used in the seed fermenter: 12.8 g/L Na2HPO4·7H2O; 5.0 g/L KH2PO4; 1.0 g/L NH4Cl; 0.5 g/L NaCl; 0.3 g/L MgSO4; 0.03 g/L CaCl2; 2.6 g/L (NH4)2SO4; 50 mg/L FeCl3·6H2O; 1.8 mg/L ZnSO4·7H2O; 0.0 to 1.5 g/L citric acid; and 0.1 ml/L antifoam. Dextrose and thiamine were aseptically added at a concentration of 35 to 40 g/L and 0.05 g/L, respectively. Seed tank conditions at EFT=0: temperature was 37°C, pH was 7.0, agitation was 200 RPM, airflow was 1.5 vvm, and back pressure was 15 psig. During incubation, pH was controlled with 28% aqueous NH4OH at set point of 7.00 and dissolved oxygen was controlled at 35% using agitation (max 500 RPM). The culture incubated until OD660 was 25 to 30 units.

When OD660 reached 25 to 30 units in the seed fermenter, the culture was transferred to a production fermenter at a 2.5% ratio. Media was used in the production fermenter: 2.00 g/L (NH4)2SO4; 8.00 g/L K2HPO4; 2.00 g/L NaCl; 1.00 g/L Na3Citrate·2H2O; 1.00 g/L MgSO4·7H2O; 0.03 g/L CaCl2·2H2O; 0.05 g/L FeSO4·7H2O; 0.0 to 1.5 g/L citric acid; 0.01 ml/L antifoam; 1.20 to 1.80 g/L yeast extract; and 0.40 ml/L Neidhardt micronutrients solution. The Neidhardt micronutrients solution consisted of: 0.18 g/L (NH4)6(MO7)24·4H2O; 1.24 g/L H3BO3; 0.36 g/L CoCl2·6H2O; 0.12 g/L CuSO4·5H2O; 0.80 g/L MnCl2·4H2O; and 0.14 g/L ZnSO4·7H2O. Dextrose and thiamine were aseptically added at a concentration of 5.0 g/L and 0.05 g/L, respectively. Production tank conditions at EFT=0: temperature was 37°C, pH was 7.0, agitation was 100 RPM, airflow was 1.5 vvm, and back pressure was 15 psig. During growth phase, pH was controlled with 28% aqueous NH4OH at set point of 7.00, dissolved oxygen was controlled at 35% using agitation (max 340 RPM), and dextrose concentration was maintained between 2 and 10 g/L. The growth phase lasted until OD660 was 25 to 30 units.

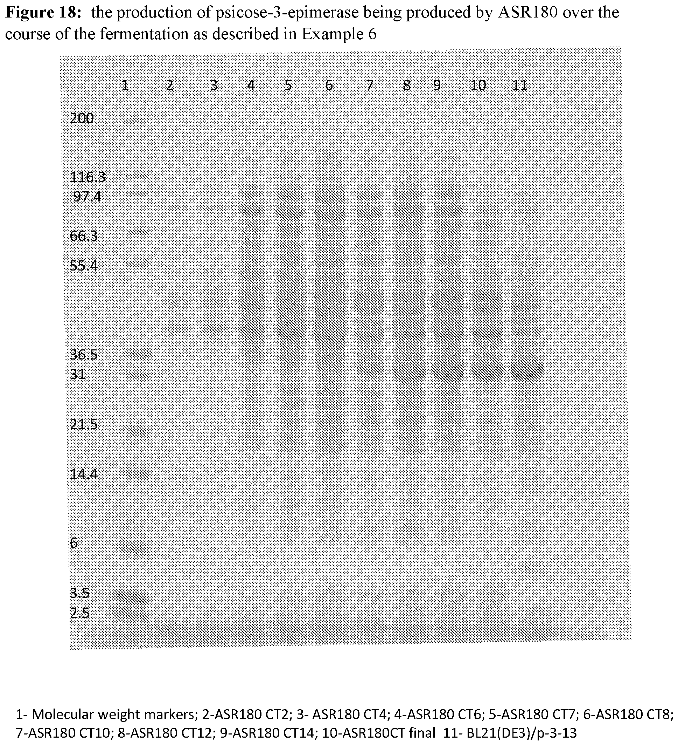

Once the OD 660 reached 25 to 30 units in the production fermenter, induction phase began. A bolus of 5.00 g/L lactose or 0.8 mMol/L IPTG was added and the temperature was linearly ramped down from 37°C to 30°C over the course of 1 hour. Production tank conditions after induction: temperature was 30°C, pH was 7.0, agitation was 340 RPM, airflow was 1.5 vvm, and back pressure was 15 psig. During induction phase, pH was controlled with 28% aqueous NH4OH at set point of 7.00, dissolved oxygen was "as is," and dextrose concentration was maintained less than 1.0 g/L. The results of the enzyme specific activity over the course of the pilot scale fermentation can be seen in Figure 8. In addition, Figure 18 shows the production of psicose-3-epimerase being produced by ASR180 over the course of this fermentation.

At the end of fermentation, the fermentation broth was processed by sodium phosphate salts being added to a final concentration of 50 mM phosphate and pH of 7.5. The fermentation broth was passed through a homogenizer (Panda NS1001L 2K from Niro-Soavi, Inc.) at 800-1000 bar, 4°C to disrupt the cells. The crude lysate was clarified by centrifugation followed by filtration with diatomaceous earth to remove any cell debris. The supernatant was used for immobilization.

Dow resin DUOLITE^{™} A568 was used for immobilization of the enzyme. Prior to immobilization, the resin was regenerated per manufacture's protocol and washed with 50 mM phosphate buffer with 2 mM MgSo4, pH 7.5. It was determined that a protein load of 100 mg/ g of dry resin was optimal for getting maximum enzyme immobilization. A jacketed glass column (Ace glass #15 15 mmX 450 mm) was filled with a known volume of resin. A known volume of crude lysate was recirculated through the resin bed at 2-4 BV/hr for 4 hr. The resin bed was then washed with 10 bed volumes of 50 mM phosphate buffer with 2 mM MgSo4, pH 7.5 to remove any unbound protein. The resin again was washed with 1:1(v/v) Glycerol: 50 mM phosphate buffer with 2 mM MgSo4, pH 7.5 and stored for future use. The results of this can be seen in Figure 18. As seen in the gel data, the binding of the psicose-3-epimerase enzyme to DUOLITE^{™} A568 is very specific. The 32 kd band for psicose-3-epimerase is not observed in the eluate fraction (lane 3) or in the subsequent wash fraction. The psicose-3-epimerase activity is observed in the resin and no activity is observed in the eluate fraction or wash fraction.

### Example 7: Conversion of fructose to allulose using psicose-3-epimerase from Burkholderia sp. RPE4 immobilized on Dow resin DUOLITE^{™} A568 resin

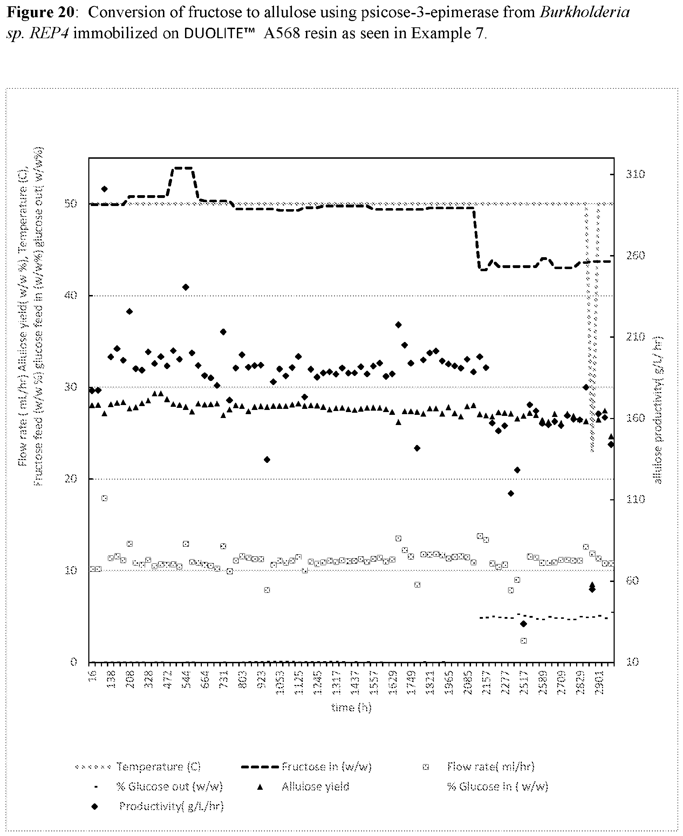

The reaction were carried out using feed consisting of solubilized crystalline fructose (50% w/w) or high fructose com syrup 90(50% DS), magnesium Mg+2 concentration of 24-50 ppm, pH range of 7.7 -6.5. The flow rate was adjusted to 1 bed volume per hour and temperature of the column was maintained at 50°C. Results can be seen in Figure 20. Under these conditions, near equilibrium production of allulose was observed for 3000 hr with minimum loss in performance, which was surprising for this resin. As described by Woodyer *et al.,* an epimerase enzyme that was immobilized on the DUOLITE^{™} A568 resin showed a decrease in the percentage conversion of fructose to allulose over the course of as little as 8 hours when the reaction was maintained at 53°C.

### Example 8: Fructose to allulose conversion using Tagatose-3-epimerse from Burkholderia Sp. RPE4 immobilized on Lifetech^{™} ECR8415 resin.

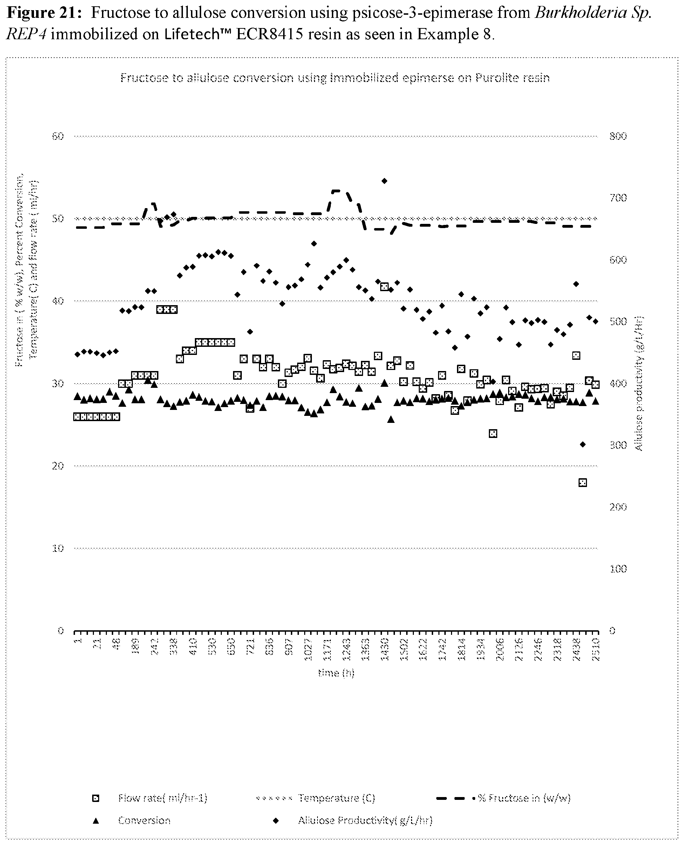

The reaction were carried out using feed consisting of solubilized crystalline fructose (50% w/w) or high fructose com syrup 90(50% DS), Mg+2 concentration of 24-50 ppm, pH range of 7.7 -6.5. The flow rate was adjusted to 3-4 bed volume per hour and temperature of the column was maintained at 50°C. Under these conditions, near equilibrium production of allulose was observed for ~3000 hr with minimum loss in performance. Results can be seen in the chart in Figure 21.

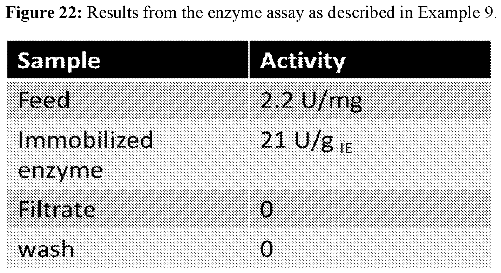

**Example 9:** Enzyme assay for fermentation samples: The fermentation samples were stored at 4°C until processed. Fermentation samples (1mL) was centrifuged and supernatant was discarded. The cell pellet was lysed using Bugbuster^{®} (EMD Millipore), centrifuged and the supernatant was used for the enzyme assay. The psicose-3-epimerase enzyme assays were performed at 50°C for 5 min in 3% (w/w) fructose and cell lysate (1:10 dilution v/v) of crude lysate. The reactions were stopped by adjusting the pH to 2 with 5% (v/v) HC1. The reaction mixtures were analyzed by HPLC. One unit of D-psicose-3-epimerase activity was defined as the amount of the enzyme required to produce 1 µmol of psicose per min at pH 7.5 and 50°C. These results seen in Figure 22.

**Example 10:** Enzyme assay for immobilized enzyme: A 10 g solution of 50 % (w/w) fructose containing feed consisting of crystalline fructose (50 % w/w) or high fructose corn syrup 90(50% DS), magnesium( Mg ⁺²) concentration of 24-50 ppm, pH range of 7.75. 1 g of immobilized enzyme was added to this reaction mixture and incubated in an orbital shaker at 50°C for 60 min. The moisture content of immobilized enzyme was measured and accounted for in the enzyme reaction. The reactions were terminated by adjusting the pH to 2 with 5 % HCL (v/v). The reaction mixture were analyzed by HPLC. One unit of D-psicose-3-epimerase activity was defined as the amount of the immobilized enzyme resin required to produce 1 µmol of psicose per min at pH 7.75 and 50 °C.
A method of producing allulose comprising: contacting a solution containing fructose with a psicose-3-epimerase enzyme from a *Burkholderia* species having at least 84% sequence identity to SEQ ID NO: 1 for a time and under conditions suitable to convert at least a portion of the fructose to allulose.
The method as defined above, wherein said psicose-3-epimerase is immobilized on a solid matrix resin. The method as defined above, wherein said solid matrix resin is a weak base anion exchange resin. The method as defined above, wherein said solid matrix resin is a phenol formaldehyde based condensate resin functionalized to contain tertiary amine free base groups.
The method as defined above, wherein said solid matrix resin is DUOLITE^{™} A568. The method as defined above, wherein said solid matrix is a methacrylic acid based resin functionalized to contain C2-C6 amine linkages.
The method as defined above, wherein said solid matrix resin from the group consisting of Lifetech^{™} ECR8315, Lifetech^{™} ECR 8415, and SEPABEADS^{™} EC -HA. The method as defined above, wherein said resin is Lifetech^{™} ECR 8415.
The method as defined above, wherein said *Bulkholderia sp.* is selected from the group consisting of *Burkholderia RP64, Candidatus Burkholderia verschuerenii, Burkholderia jiangsuensis, Burkholderia sp. MR1, Burkholderia grimmiae,* and *Candidatus Burkholderia brachyanthoides.*
The method as defined above, wherein said *Bulkholderia sp.* is *Burkholderia RP64.* The method as defined above, wherein said enzyme has a polypeptide sequence selected from the group consisting of SEQ ID NO: 1, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO:20, SEQ ID NO:21, SEQ ID NO:22, SEQ ID NO:23, SEQ ID NO:24, SEQ ID NO:25, and SEQ ID NO:26.
The method as defined above, wherein the conversion of fructose to allulose is done at a temperature of between 50 °C and 70 °C.
The method as defined above, wherein said fructose solution is selected from the group consisting of solubilized crystalline fructose and high fructose corn syrup (HFCS).
The method as defined above, wherein said fructose solution has a dissolved solids content of about 50 %w/w, the contacting is done at a pH range of 6.5-7.7 in the presence of magnesium at a concentration of 24-50 ppm, and is done with a flow rate of 1-4 volumes of fructose solution per bed volume of the solid matrix resin. The method as defined above, wherein said psicose-3-epimirase enzyme is obtained from a microorganism containing a recombinant nucleic acid vector operably configured to express a nucleic acid sequence encoding the protein having at least 84 % sequence identity to SEQ ID NO: 1.
The method as defined above, wherein said microorganism is selected from the group consisting of *E. coli* and *B. subtillis.*
A recombinant nucleic acid sequence operably configured to express a nucleic acid sequence encoding a protein having at least 84% sequence identity to SEQ ID NO: 1 in a microorganism.
A microorganism transformed with the recombinant nucleic acid sequence as defined above.
The microorganism as defined above, wherein the microorganism is selected from the group consisting of *E. coli* and *B. subtillis.*
A solid matrix resin containing a psicose-3-epimerase enzyme having at least 84% sequence identity to SEQ ID NO: I immobilized thereon.
A column containing the solid matrix resin according to claim 20 and configured to receive an input flow of a solution to flow over the solid matrix resin and permit exit of an output flow of the solution.

## Claims

1. A method of producing allulose comprising:
contacting a solution containing fructose with a psicose-3-epimerase enzyme from a *Burkholderia* species having at least 84% sequence identity to SEQ ID NO: 1 for a time and under conditions suitable to convert at least a portion of the fructose to allulose.

2. The method of claim 1, wherein said psicose-3-epimerase is immobilized on a solid matrix resin.

3. The method of claim 2, wherein said solid matrix resin is a weak base anion exchange resin.

4. The method of claim 2, wherein said solid matrix resin is a phenol formaldehyde based condensate resin functionalized to contain tertiary amine free base groups.

5. The method of claim 2, wherein said solid matrix is a methacrylic acid based resin functionalized to contain C2-C6 amine linkages.

6. The method of claim 1 wherein said *Bulkholderia sp.* is selected from the group consisting of *Burkholderia RP64, Candidatus Burkholderia verschuerenii, Burkholderia jiangsuensis, Burkholderia sp. MR1, Burkholderia grimmiae,* and *Candidatus Burkholderia brachyanthoides,* preferably wherein said *Bulkholderia sp.* is *Burkholderia RP64.*

7. The method of claim 1 wherein said enzyme has a polypeptide sequence selected from the group consisting of SEQ ID NO: 1, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO:20, SEQ ID NO:21, SEQ ID NO:22, SEQ ID NO:23, SEQ ID NO:24, SEQ ID NO:25, and SEQ ID NO:26.

8. The method of claim 3 wherein the conversion of fructose to allulose is done at a temperature of between 50°C and 70°C.

9. The method of claim 1, wherein said fructose solution is selected from the group consisting of solubilized crystalline fructose and high fructose corn syrup (HFCS), and preferably wherein said fructose solution has a dissolved solids content of about 50%w/w, the contacting is done at a pH range of 6.5-7.7 in the presence of magnesium at a concentration of 24-50ppm, and is done with a flow rate of 1-4 volumes of fructose solution per bed volume of the solid matrix resin.

10. The method of claim 1 wherein said psicose-3-epimirase enzyme is obtained from a microorganism containing a recombinant nucleic acid vector operably configured to express a nucleic acid sequence encoding the protein having at least 84% sequence identity to SEQ ID NO: 1, preferably wherein said microorganism is selected from the group consisting of *E. coli* and *B. subtillis.*

11. A recombinant nucleic acid sequence operably configured to express a nucleic acid sequence encoding a protein having at least 84% sequence identity to SEQ ID NO: 1 in a microorganism.

12. A microorganism transformed with the recombinant nucleic acid sequence according to claim 11.

13. The microorganism of claim 12 wherein the microorganism is selected from the group consisting of *E. coli* and *B. subtillis.*

14. A solid matrix resin containing a psicose-3-epimerase enzyme having at least 84% sequence identity to SEQ ID NO: I immobilized thereon.

15. A column containing the solid matrix resin according to claim 14 and configured to receive an input flow of a solution to flow over the solid matrix resin and permit exit of an output flow of the solution.
